# EUROPÄISCHE PATENTSCHRIFT

(11) **EP 1 080 743 B1**
(45) Veröffentlichungstag und Bekanntmachung des Hinweises auf die Patenterteilung: **28.12.2005**
(21) Anmeldenummer: 00118120.5
(22) Anmeldetag: 25.08.2000
(51) Int. Cl.: A61M 15/00

(54) **Inhaliergerät**
Inhaler
Inhalateur

(30) Priorität: 03.09.1999 DE 19942080
(43) Veröffentlichungstag der Anmeldung: 07.03.2001
(73) Patentinhaber: InAMed GmbH, 35285 Gemünden (DE)
(72) Erfinder: Scheuch, Gerhard, 35285 Gemünden/Wohra (DE)
(74) Vertreter: Reinhard - Skuhra - Weise & Partner

(56) Entgegenhaltungen:
- EP-A- 0 491 426
- WO-A-00/54828
- WO-A-98/49659
- US-A- 5 363 842
- US-A- 5 855 307

## Beschreibung

Die Erfindung betrifft ein Inhaliergerät fiir Trockenpulver-Aerosole.

In der WO 00/54828 ist ein Inhaliergerät angegeben mit einem Vorratsraum zur Aufnahme des Inhaliermediums, welches unter Druck steht. Der Vorratsraum ist mit einem das Aerosol freigebenden Mechanismus verbunden, um in dosierter Weise das Aerosol bei Betätigung des Mechanismus freizugeben.

Verschiedene Patienten müssen regelmäßig ein Medikament einnehmen oder benötigen ein Notfallmedikament. In vielen Fällen ist das Medikament nicht so schnell griffbereit oder betriebsfertig, wie es erforderlich wäre. Asthmapatienten beispielsweise müssen bei den ersten Anzeichen eines Asthmaanfalles das Notfallpräparat zur Hand haben. In diesem Fall werden in der Regel Aerosole verabreicht, zu welchem Zweck entsprechende Geräte angeboten werden, nämlich der sogenannte Metered Dose Inhaler und Dry Powder Inhaler. Diese Geräte werden in der Jackentasche oder in einer Handtasche mitgeführt und sind erst nach einigen Handgriffen betriebsbereit. Nachteilig ist daher, dass ein Patient insbesondere im panikartigen Zustand in Schwierigkeiten gerät, wenn er das Gerät in Betrieb nehmen muss und es unter Umständen erst noch suchen muss.

Bei Diabetes Patienten ist die rechtzeitige Einnahme eines Medikamentes vor den Mahlzeiten erforderlich. Das Medikament wird bislang durch Spritzen, in der Zukunft auch durch Inhalation eingenommen. Wenn der Patient das Medikament jedoch vergessen hat oder sich durch sportliche Aktivitäten stark angestrengt hat, kann es vorkommen, dass er in eine Art Koma verfällt und sich nur durch die Hilfe von Angehörigen mittels Gabe von Traubenzucker wieder erholt.

Auch bei anderen Medikamenten ist die rechtzeitige Einnahme eines Wirkstoffes, zum Teil mehrmals am Tag, wichtig.

Der Erfindung liegt daher die Aufgabe zugrunde, ein tragbares Inhaliergerät für Trockenpulver-Aerosole zu schaffen, welches auf einfache und sichere Weise aktivierbar ist.

Diese Aufgabe wird erfindungsgemäß durch die Merkmale des Patentanspruchs 1 gelöst.

Weitere Ausgestaltungen des erfindungsgemäßen Inhaliergerätes ergeben sich aus den Unteransprüchen.

Das erfindungsgemäße Inhaliergerät ist vorteilhafterweise eine Kombination aus Armbanduhr und Inhaliergerät, wodurch es möglich ist, ein Medikament oder einen Wirkstoff in Form einer Armbanduhr am Unterarm mit sich zu führen oder in einer Uhr, die am Körper mitgeführt wird.

Demzufolge ist das erfindungsgemäße Inhaliergerät stets griffbereit.

Das das Inhaliergerät aufnehmende Gehäuse ist vorzugsweise das Gehäuse einer Uhr, insbesondere Armbanduhr. An dem Gehäuse ist seitlich oder unterhalb des Anzeigeblattes oder des Displays der Uhr ein Mundstück angeordnet, das ohne weiteres zum Mund geführt werden kann und im Bedarfsfall ein sofortiges Inhalieren eines Wirkstoffes oder Medikamentes ermöglicht.

Das erfindungsgemäße Inhaliergerät hat weiter den Vorteil, daß das Uhrwerk gleichzeitig als Alarmgeber verwendet werden kann, um dem Patienten die Einnahme eines Medikamentes anzuzeigen.

Weiterhin kann die Uhr mit einem Mikroflußmeßsystem ausgestattet werden, das eine Aufzeichnung des Inhalationsmanövers des Patienten und den Zeitpunkt der Inhalation registriert.

Im folgenden wird eine bevorzugte Ausführungsform des erfindungsgemäßen Inhaliergerätes an Hand der Zeichnung zur Erläuterung weiterer Merkmale und Vorteile beschrieben.

Es zeigen:
- Figur 1: eine Vorderansicht des Inhaliergerätes, und
- Figur 2: eine Schnittansicht durch das Inhaliergerät entlang der Linie II - II' in Figur 1.

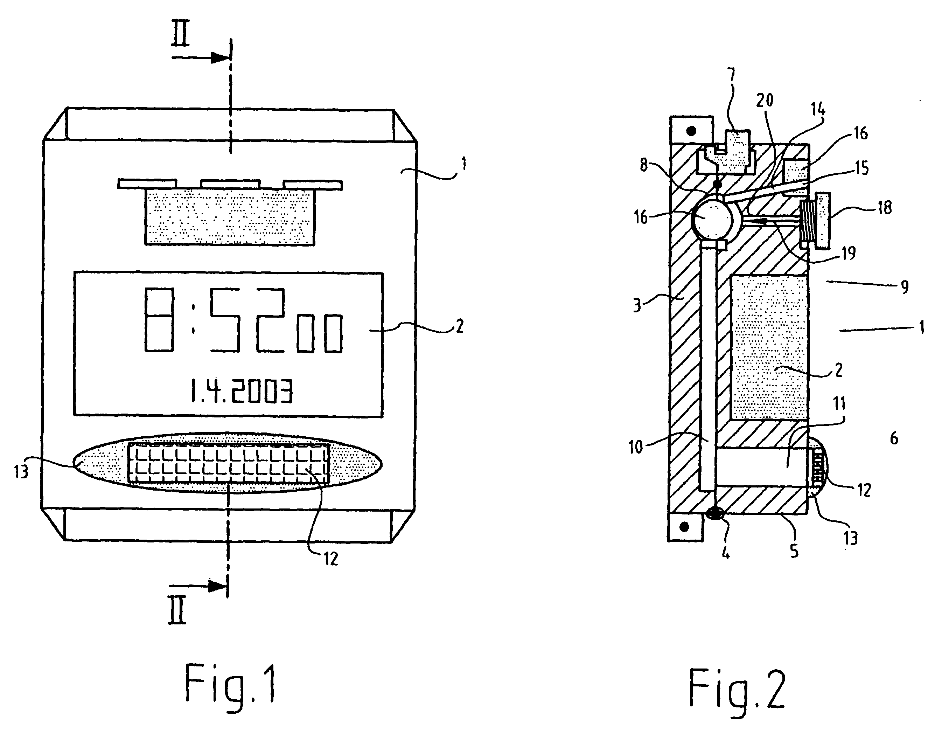

Das in den Figuren 1 und 2 gezeigte Inhaliergerät besteht aus einem Gehäuse 1, in welchem eine digitale oder analoge Uhr 2 untergebracht ist. Die Uhr 2 mit dem nicht weiter gezeigten Uhrwerk befindet sich beispielsweise mittig in dem Gehäuse 1, wie aus Figur 1 hervorgeht. Das Gehäuse 1 ist mit einem Deckel 3, beispielsweise an der Rückseite, verschlossen, wobei der Deckel 3 über ein Gelenk 4 vorzugsweise an der Unterseite des Gehäuses, die in Figur 2 mit 5 bezeichnet ist, an dem Gehäuse 1 angelenkt ist. Der Deckel 3 wird durch eine Verschlußeinrichtung 7 entriegelt, wenn die Verschlußeinrichtung 7 entsprechend aktiviert wird.

In dem Gehäuse 1 befindet sich ein Vorratsraum 8, der beispielsweise zylindrisch ausgebildet ist, wobei die Achse des zylindrischen Vorratsraumes 8 vorzugsweise parallel liegt zu der mit 9 bezeichneten Fontfläche. Vom Vorratsraum 8 geht ein Verbindungskanal 10 ab, der bei der dargestellten Ausführungsform nach Figur 2 etwa senkrecht nach unten verläuft und in einen quer hierzu stehenden weiteren Verbindungskanalabschnitt 11 übergeht. Der Verbindungskanalabschnitt 11 endet an einem Mundstück 6, welches auf die Frontflächen 9 im Bereich des Verbindungskanalabschnittes 11 aufgesetzt ist. Das Mundstück 6 ist mit einem Netzgitter 12 versehen und mit einer Gummilippe 13 abgeschlossen.

Der Vorratsraum 8 ist weiterhin über mindestens einen Eintrittskanal 14 zur Umgebung geöffnet. Die mit 15 bezeichnete Öffnung des Kanals 14 kann durch eine weitere Gummilippe 16 zumindest teilweise verschlossen sein. Die Gummilippen 13, 16 können so ausgebildet sein, daß der Patient eine minimale Flußrate erreichen muß, bevor das im Vorratsraum 8 befindliche Medikament oder dergleichen sich entleert und es ist auch weiterhin eine Fluß-Limitierung möglich, damit der Patient nicht so schnell inhaliert, daß der Wirkstoff hauptsächlich im Mund-Rachenraum deponiert wird.

Wie sich aus der Zeichnung ergibt, ist der Verbindungskanal 10, 11 gegenüber der Uhr bzw. dem Uhrwerk versetzt ausgebildet.

Gemäß einer bevorzugten Ausführungsform wird das Medikament in Form einer Kapsel 16 in den Vorratsraum 8 dadurch eingelegt, daß der Deckel 3 gegenüber dem Gehäuse 1 geöffnet bzw. verschwenkt wird, wodurch der Vorratsraum 8 freigegeben wird. Nach dem Einbringen der Kapsel 16 wird der Deckel 3 geschlossen und der Deckel wird durch die Verriegelungseinrichtung 7 im geschlossenen Zustand gehalten. Bei dieser Ausführungsform ist vorgesehen, daß durch einen Taster 18 eine Spitze bzw. Nadel in Richtung auf die Kapsel verschoben wird, um diese zur Freigabe des Wirkstoffes zu durchsetzen. Bei der Ausführungsform nach Figur 2 sitzt der Taster 18 auf bzw. an der Frontfläche 9 und ist federvorgespannt. Der Taster 18 steht mit einer Nadel oder Spitze 19 in mechanischer Verbindung, die ihrerseits in einem Führungskanal 20 verlagerbar angeordnet ist und durch die Betätigung des Tasters 18 aus dem Führungskanal 20 in Richtung auf die Kapsel 16 verstellt wird. Der Führungskanal 20 ist bei der Ausführungsform nach Figur 2 so ausgebildet, daß die Spitze 19 etwa in Richtung Mittelachse der Kapsel 16 durch die Betätigung des Tasters 18 verschoben wird.

Um den Wirkstoff freizugeben, muß der Patient somit lediglich kurzfristig den Taster 18 betätigen, um dann das Gehäuse 1 zum Mund zu führen, um das Mundstück 6 mit dem Mund zu erfassen. Damit ist es dem Patienten möglich, beinahe unauffällig das Medikament zu inhalieren.

Die enge Kanalführung der Verbindungskanäle 10, 11 ermöglicht eine gute Zerstäubung des Medikaments.

## Patentansprüche

1. Inhaliergerät für Trockenpulver-Aerosole,
mit einem in einem Uhrengehäuse angeordneten Vorratsraum (8), der zur Aufnahme einer das Trockenpulver-Inhaliermedium enthaltenen Kapsel (16) ausgelegt ist, mit einem Verbindungskanal (10, 11), der vom Vorratsraum (8) zu einem Mundstück (6) reicht und gegenüber einem Uhrwerk (2) versetzt vorbeigeführt ist, und mit einem weiteren vom Vorratsraum (8) zur Gehäuseoberfläche verlaufenden Luftkanal (14), wobei ein den Vorratsraum (8) abschließender und zum Zwecke des Einbringens der Kapsel (16) öffnungsfähiger Deckel (3) am Uhrengehäuse (1) angeordnet ist.

2. Inhaliergerät nach Anspruch 1, **dadurch gekennzeichnet, dass** das Mundstück (6) auf bzw. an einer Oberfläche (9) des Uhrengehäuses (1) vorgesehen ist.

3. Inhaliergerät nach einem der vorhergehenden Ansprüche, **dadurch gekennzeichnet, dass** eine Öffnereinrichtung (18, 19) vorgesehen ist, die einen in den Vorratsraum (8) hineinverlagerbaren Öffner (19) aufweist.

4. Inhaliergerät nach Anspruch 3, **dadurch gekennzeichnet, dass** die Öffnereinrichtung (18, 19) einen Taster (18) aufweist, der auf der Gehäuseoberfläche (9) angeordnet ist und der mit einer Nadel (9) oder dergleichen in mechanischer Verbindung steht, welche innerhalb eines Führungskanales (20) verstellbar aufgenommen ist.

5. Inhaliergerät nach Anspurch 4, **dadurch gekennzeichnet, dass** der Führungskanal (20) in den Vorratsraum (8) mündet.

6. Inhaliergerät nach wenigstens einem der vorhergehenden Ansprüche, **dadurch gekennzeichnet, dass** dem Deckel (3) eine Verschlusseinrichtung (7) zugeordnet ist.

## Claims

1. Inhaler for dry powder aerosols,
comprising a storage room (8) arranged in the case of a watch, adapted to receive a capsule (16) containing the dry powder inhaling medium, with a connecting channel (10, 11) reaching from the storage room (8) to a mouthpiece (6) and guided past a watch movement (2) in a displaced manner, and with another air duct (14) extending from the storage room (8) to the upper surface of the case, wherein a lid (3) closing the storage room (8) and openable for the purpose of introducing the capsule (16) is arranged on the case of the watch (1).

2. Inhaler according to claim 1, **characterized in that** the mouthpiece (6) is provided on or at an upper surface (9) of the case of the watch (1).

3. Inhaler according to one of the preceding claims, **characterized in that** an opening device (18, 19) is provided which comprises an opener (19) displaceable into the storage room (8).

4. Inhaler according to claim 3, **characterized in that** the opening device (18, 19) comprises a push-button (18) arranged on the upper surface (9) of the case and mechanically connected to a needle (19) or the like, which is adjustably received inside a guide channel (20).

5. Inhaler according to claim 4, **characterized in that** the guide channel (20) opens into the storage room (8).

6. Inhaler according to one of the preceding claims, **characterized in that** a closing device (7) is assigned to the lid (3).

## Revendications

1. Appareil d'inhalation pour des aérosols à poudre sèche,
comportant un réservoir (8) disposé dans un boîtier de montre qui est conçu pour loger une capsule (16) contenant un milieu d'inhalation à poudre sèche, comportant un conduit de liaison (10,11) qui s'étend du réservoir (8) à une embouchure (6) et disposé d'une manière décalée par rapport à un mouvement de montre (2), et comportant un autre conduit d'air (14) qui s'étend depuis le réservoir (8) jusqu'à la surface du boîtier, le couvercle (3), qui ferme le réservoir (8) et peut s'ouvrir pour l'introduction de la capsule (16), étant disposé dans le boîtier de montre.

2. Appareil d'inhalation selon la revendication 1, **caractérisé en ce que** l'embouchure (6) est prévue sur ou au niveau d'une surface (9) du boîtier de montre (1).

3. Appareil d'inhalation selon l'une des revendications précédentes, **caractérisé en ce qu'**il est prévu un dispositif ouvreur (18,19), qui comporte un système ouvreur (9) pouvant être décalé en retrait dans le réservoir (8).

4. Appareil d'inhalation selon la revendication 3, **caractérisé en ce que** le dispositif ouvreur (18,19) comporte une touche (18) qui est disposée sur la surface (9) du boîtier et est reliée mécaniquement à une aiguille (9) ou analogue, qui est logée de manière à être déplaçable à l'intérieur d'un conduit de guidage (20).

5. Appareil d'inhalation selon la revendication 4, **caractérisé en ce que** le conduit de guidage (20) débouche dans le réservoir (8).

6. Appareil d'inhalation selon au moins l'une des revendications précédentes, **caractérisé en ce qu'**un dispositif de fermeture (17) est associé au couvercle (3).
